# EUROPEAN PATENT SPECIFICATION

(11) **EP 1 867 991 B1**
(45) Date of publication and mention of the grant of the patent: **14.09.2011**
(21) Application number: 06781487.1
(22) Date of filing: 24.07.2006
(51) Int. Cl.: G01N 33/543, G01N 33/569

(54) **IMMUNOASSAY METHOD AND IMMUNOASSAY KIT TO BE USED THEREIN**
IMMUNOASSAY-VERFAHREN UND DABEI ZU VERWENDENDER KIT
PROCEDE DE TEST IMMUNITAIRE ET KIT DE TEST IMMUNITAIRE A UTILISER DANS CELUI-CI

(30) Priority: 25.07.2005 JP 2005214895
(43) Date of publication of application: 19.12.2007
(73) Proprietor: ARKRAY, Inc., Kyoto-shi, Kyoto 601-8045 (JP)
(72) Inventor: OHMIYA, Kazuhiro, Kyoto-shi, Kyoto 601-8045 (JP); OHSHIRO, Kyouichi, Kyoto-shi, Kyoto 601-8045 (JP)
(74) Representative: Matthews, Derek Peter
(86) International application number: PCT/JP2006/314575
(87) International publication number: WO 2007/013401

(56) References cited:
- EP-A- 0 818 680
- JP-A- 11 248 709
- JP-A- 55 087 048
- JP-A- 2001 318 101
- US-A- 4 270 923
- MOLINA-BOLIVAR J A ET AL: "Particle enhanced immunoassays stabilized by hydration forces: a comparative study between IgG and F(ab')2 immunoreactivity" JOURNAL OF IMMUNOLOGICAL METHODS, ELSEVIER SCIENCE PUBLISHERS B.V.,AMSTERDAM, NL, vol. 211, no. 1-2, 1998, pages 87-95, XP004120570 ISSN: 0022-1759

## Description

### Technical Field

The present invention relates to an immunoassay method and an immunoassay kit used in the same.

### Background Art

Recently, simple testing reagents and kits that enable detection or quantification of measurement items by utilizing an antigen-antibody reaction within several minutes to several tens of minutes from the starting of the measurement have been developed. In the case where the measurement item is an infectious disease, if infection or noninfection of the specimen that is collected from the patient is judged instantly, the patient can be treated with medication while the condition is at an earlier stage. Thus, the importance of such simple testing reagents and kits have increased year by year in the field of medical treatment.

Presently, as one of the simple testing methods, an immunoassay method such as a latex agglutination assay is used widely. The latex agglutination assay is a method in which an antibody-sensitized latex particle and a target antigen in the specimen are bound and agglutinated by the antigen-antibody reaction so as to detect the infection or the noninfection according to a degree of the agglutination. In this method, depending on a concentration of the specimen, the agglutination can be visually judged, and the quantitative test by the measurement of the absorbance with an automatic analyzing apparatus should be done.

However, in the case of analyzing a specimen (for example, a throat swab or the like) by using the immunoassay method such as the latex agglutination assay, there is a problem in that false positives occur. That is, since the particles are bound and agglutinated due to a nonspecific reaction regardless of the absence of the analyte in the specimen, it is decided to be positive. If the false positives occur as described above when specifying the infection of the pathogen, incorrect information is given regarding the affection. This may result in the delay of specifying the cause and inappropriate treatment, which may cause serious problems such as a more severe condition of the disease and the like. Thus, the suppressing of the occurrence of the false positives is an extremely important problem.

Conventionally, in order to solve this problem, a method for suppressing the occurrence of the false positives by using a filter or the like in advance so as to screen the specimen according to a mesh size of the filter is suggested (for example, in Patent document 1). However, this method achieves its effect in EIA (enzyme immunoassay) using a membrane solid phase, but cannot provide a sufficient effect in the immunoassay method using antibody sensitization particles such as the latex agglutination assay.
Patent document 1: JP 2004-28875 A

### Disclosure of Invention

### Problem to be Solved by the Invention

Then, it is an object of the present invention is to provide an immunoassay method that prevents the occurrence of false positives and has a high detecting precision and a high quantification precision in the immunoassay method using antibody sensitization particles, and an immunoassay kit used in the same.

### Means for Solving Problem

In order to attain the above-described objects, the immunoassay method of the present invention is an immunoassay method for detecting or quantifying an analyte in a specimen, including: a step of mixing the specimen with an antibody sensitization particle obtained by sensitizing a particle with an antibody that reacts with the analyte in the specimen; a step of measuring agglutination of the antibody sensitization particle by an antigen-antibody reaction; and a step of pre-treating the specimen by using a cation exchange material in advance of the mixing step.

Moreover, the immunoassay kit of the present invention is an immunoassay kit that is used in the immunoassay method of the present invention, and includes: an antibody sensitization particle obtained by sensitizing a particle with an antibody that reacts with an analyte in a specimen; and a cation exchange material.

### Effects of the Invention

As described above, if the specimen is pre-treated by using the cation exchange material, a non-specific reaction can be prevented. Thus, the immunoassay method that can suppress the occurrence of false positives and has a high detecting precision and a high quantification precision can be achieved.

### Description of the Invention

Next, the present invention will be described in detail.

The cation exchange material is not limited particularly, but examples thereof include a strong acid cation exchange material and a weak acid cation exchange material, and among them, a cation exchange material having a sulfonic acid group or a carboxyl group as a functional group is preferable. Moreover, the cation exchange material may have both of a sulfonic acid group and a carboxyl group as the functional group.

As the cation exchange material, a cation exchange resin is exemplified. In particular, a resin having at least one of a sulfonic acid group and a carboxyl group as the functional group is preferable. Specific examples of the cation exchange resin include: polyethersulfone; polysulfone; a resin obtained by introducing at least one of a sulfonic acid group and a carboxyl group into a polystyrene-divinylbenzene copolymer; a resin obtained by introducing at least one of a sulfonic acid group and a carboxyl group into a hydrophilic methacrylate-based polymer; and the like. Among them, polyethersulfone and a resin obtained by introducing at least one of a sulfonic acid group and a carboxyl group into a hydrophilic methacrylate-based polymer are preferable. A shape of the cation exchange resin is not limited particularly, and may be, for example, a spherical shape, a film, a fiber and the like.

A form of the cation exchange material is not limited particularly, but is preferably, for example, a cation exchange filter, a cation exchange column and the like. Examples of the cation exchange filter include a filter in which a spherical-shaped cation exchange resin is supported by a carrier, a filter containing a fiber-shaped cation exchange resin and the like. A material of the carrier is preferably, for example, a material other than the cation exchange resin, such as cotton, and a shape of the carrier is preferably fiber-shaped. Examples of the filter in which the cation exchange resin is supported by the carrier include a filter in which a powder polyethersulfone is supported by absorbent cotton and the like. Moreover, examples of the filter containing the fiber-shaped cation exchange resin include a filter made of a knit polyethersulfone fiber and the like. Examples of the cation exchange column include a tube that is filled with the cation exchange resin and the like.

The antibody that reacts with the analyte is not limited particularly, but may be, for example, a polyclonal antibody, a monoclonal antibody or the like that reacts and binds specifically to the analyte.

As the antibody sensitization particle, for example, a particle that is sensitized with the antibody can be used. The particle is not limited particularly, but may be an insoluble carrier particle, and examples of the insoluble carrier particle include: a latex particle such as a polystyrene latex particle; a polypropylene particle; a polyethylene particle; a gelatin particle; a metal colloid; and the like, and among them, a polystyrene latex particle is preferable. The antibody sensitization particle is preferably an antibody sensitization latex particle that is obtained by sensitizing the polystyrene latex particle with an antibody. Incidentally, the particle with the antibody can be sensitized by a conventionally known method.

Moreover, in the immunoassay method of the present invention, assembly that is provided with the antibody sensitization particle may be used. Examples of the assembly include a reagent pack containing a reagent and the like, and the reagent preferably includes the antibody sensitization particle. The reagent pack preferably has, for example, a disposable cartridge form, and more preferably, includes two or more vessels, and has a reagent vessel containing the reagent as at least one of the vessels. The reagent pack preferably includes, in addition to the reagent vessel, a reaction vessel for undergoing the reaction, a specimen vessel for containing the specimen, a waste vessel for containing a waste liquid, a dispensing tip and the like, for example. By using the reagent pack provided with such vessels, for example, preparation of the reagent and the like are not necessary, and thus the pre-treatment and the measurement of the specimen can be carried out continuously. As a result, the measurement can be simplified more, and an application thereof to an automated apparatus can be significantly easier. As the reagent pack, for example, a cartridge for immunoassay described in JP 2001-318101 A can be used. The cartridge includes, for example, the reagent vessel and the reaction vessel, and further may include the specimen vessel, the waste vessel, the dispensing tip and the like. The numbers of the reagent vessels and the reaction vessels are not limited particularly, and may be one or more. Disposition of these vessels is not limited particularly, but for example, in the case where a plurality of the reagent vessels and the reaction vessels are provided respectively, and are disposed in series, the reagent vessels and the reaction vessels preferably are disposed alternately. Moreover, an upper portion of each of the reagent vessels and the reaction vessels preferably is sealed with, for example, an aluminum foil, a polymer film or the like. As the reagent, for example, a reaction buffer solution and the like that will be described below can be used.

Examples of the immunoassay method used in the present invention include a latex agglutination assay. The present invention preferably is applied to the latex agglutination assay.

Examples of the analyte include a bacterium, a virus and the like. In the immunoassay method of the present invention, by detecting or quantifying a specific antigen of the bacterium or the virus, the bacterium or the virus can be detected or quantified. Specific examples of the bacterium and the virus include: a hemolytic streptococcus; an influenza virus; an adenovirus; a RS (Respiratory Syncytial) virus; a Hemophilus pertussis; a chlamydia trachomatous; a coronavirus (SARS); a mycoplasma; and the like. Among them, the immunoassay method of the present invention is useful for a hemolytic streptococcus and an influenza virus.

The specimen is not limited particularly, but examples thereof include a specimen from nasal secretion, a specimen from throat secretion and the like. Examples of the specimen from nasal secretion include a nasal swab, a nasal aspirate, a nasal wash and the like. Moreover, examples of the specimen from throat secretion include a throat swab, saliva and the like.

Next, the immunoassay kit of the present invention is an immunoassay kit used in the immunoassay method of the present invention as described above, and includes: an antibody sensitization particle obtained by sensitizing a particle with an antibody that reacts with an analyte in the specimen; and a cation exchange material.

The immunoassay kit of the present invention further may include a reagent pack containing a reagent, and the reagent preferably includes an antibody sensitization particle. The reagent pack preferably has, for example, a disposable cartridge form, and more preferably includes two or more vessels and has the reagent vessel containing the reagent as the vessel. Further, the reagent pack preferably includes a reaction vessel, a specimen vessel, a dispensing tip, a waste vessel and the like. In the case where the immunoassay kit of the present invention includes the reagent pack that is provided with a plurality of the vessels, the immunoassay method of the present invention can be carried out more simply, and its application to an automated apparatus can be significantly easier.

In order to improve its convenience more, it is preferable that the immunoassay kit of the present invention further includes, for example, at least one of: a specimen diluent; a reaction buffer solution; a specimen collecting tool; an specimen extract solution; and the like.

Examples of the immunoassay kit of the present invention include the cartridge for immunoassay in which the antibody sensitization particle, a cation exchange material and at least one of: the specimen diluent; the reaction buffer solution; the specimen collecting tool; and the specimen extract solution are enclosed as appropriate.

The specimen diluent is a reagent for diluting the specimen before the measurement, and may be determined as appropriate according to the analyte, the specimen and the like, for example. The specimen diluent is not limited particularly, but examples thereof include PBS containing BSA and NaN₃, and the like. It should be noted that the specimen diluent may be omitted depending on a kind, an amount and the like of the specimen.

The reaction buffer solution can be determined as appropriate, for example, according to the analyte and the like, and is not limited particularly, but examples thereof include a Tris-HCl buffer solution, a phosphate buffer solution and the like. The reaction buffer solution may contain, for example, polyethylene glycol (PEG20000 or the like), BSA (bovine serum albumin), NaCl, EDTA-2Na, NaN₃ and the like.

The specimen collecting tool may be, for example, the dispensing tip that functions also as the specimen collecting tool, a cotton bud or the like.

The specimen extract solution is, for example, a reagent that is used for extracting a nucleoprotein and the like, which can be determined as appropriate according to the analyte, the specimen and the like, and is not limited particularly, but for example, a buffer solution or the like can be used. Examples of the buffer solution include: a CHES
(N-Cyclohexyl-2-aminoethanesulfonic acid) buffer solution; a phosphate buffer solution; a Tris-HCl buffer solution; a CAPS
(N-Cyclohexyl-3-aminopropanesulfonic acid) buffer solution; and the like. The specimen extract solution may contain: semialkali protease; n-Octanol-N-methylglucamide; BSA; NaCl; CaCl₂; EDTA-2Na; NaN₃; and the like, for example.

An example of the measurement method of the present invention will be described below, but the present invention is not limited to the method described below. A specimen from throat secretion is used as the specimen, and a case where the analyte is a hemolytic streptococcus is exemplified for explanation.

A cation exchange material is provided. As the cation exchange material, the above-described materials can be used, but for example, a cation exchange filter that is disposed between glass filters, which is filled inside a nozzle made of polyethylene or the like is preferable. Moreover, as the cation exchange material, a commercially available cation exchange filter and cation exchange column may be used.

Next, the specimen from throat secretion that is collected from the throat is mixed with, for example, a 2 M sodium nitrite solution and a 1M acetic acid solution, and subsequently is neutralized so as to prepare a specimen solution. In the case where a specimen amount is very small, the specimen may be suspended in advance with a specimen diluent such as PBS (pH 7.4) containing 1 wt% BSA and 0.1 wt% NaN₃, for example.

And, the specimen solution is subjected to cation exchange treatment by using the cation exchange material. Thereafter, the specimen solution after the treatment, an antihemolytic streptococcus antibody sensitization latex reagent and the reaction buffer solution are mixed, and a degree of its agglutination is detected. The degree of the agglutination may be detected by, for example, suspension measurement using an optical measurement apparatus that is controlled automatically, such as a spectrophotometer, and visual observation of the presence or absence of the agglutination on a slide glass or the like. The antihemolytic streptococcus antibody sensitization latex reagent that is prepared by the user or a commercially available one may be used.

Next, a case where the analyte is an influenza virus will be exemplified for explanation.

It is preferable that the influenza virus is subjected to screening so as to be determined to be, for example, a type A or a type B by the immunoassay method of the present invention. An antibody used for the screening is preferably an antibody that is specific to the nucleoprotein, for example. Since the antigenicity of hemagglutinin or the like of a surface protein of the influenza virus is mutated one after another, if using a certain antihemagglutinin antibody, the reactivity differs according to a subtype of the epidemic virus, and there is a possibility that the reaction does not occur in some cases. However, the nucleoprotein maintains the antigenicity that is a basis when classifying into the types A and B, and exhibits certain reactivity regardless of its subtype, thereby being suitable for screening to classify into the types A and B. Since the nucleoprotein is localized inside an envelope that is composed of a lipid bilayer membrane of the virus particle, and does not exist on a surface of the virus, it is preferable to extract the nucleoprotein by, for example, physically breaking of the virus particle by ultrasonic treatment, repeating of freezing and thawing, and the like, and by a chemical treatment method using a surfactant and the like. Examples of the chemical treatment method includes a method for extracting by a nonionic surfactant such as Tween20 (product name, generic name: Polyoxyethylene Sorbitan Monolaurate) and n-Octanoyl-N-methylglucamide (for example, product name: MEGA-8). In an EIA method, immunochromatography and the like, the Tween20 and the like are commonly used as the surfactant, but in the case of undergoing the antigen-antibody reaction by using the agglutination of the antibody sensitization particle like the present invention, n-Octanoyl-N-methylglucamide (for example, product name: MEGA-8) is preferably used, because of its small influence on the reactivity of the agglutination reaction of the particle and its high extracting effect. Subsequently, it is preferable to carry out the screening by using the thus extracted nucleoprotein by the immunoassay method of the present invention.

Next, examples of the present invention will be described with comparative examples. However, the present invention is not limited to the examples described below.

### Example 1

### 1. Preparation of antihemolytic streptococcus antibody sensitization latex reagent

An antibody solution (antibody concentration of 0.7 mg/mL) was prepared by using an antihemolytic streptococcus rabbit polyclonal antibody in 50 mM boric acid buffer solution (pH 7.4). 0.5 mL of the antibody solution and of 0.5 mL of 1% (w/v) polystyrene latex particles (produced by SEKISUI CHEMICAL CO., LTD, average particle diameter of 0.5 µm) were mixed, and were incubated at 37°C for 1 hour so as to sensitize the polystyrene latex particles with the antihemolytic streptococcus rabbit polyclonal antibody. Thereafter, BSA (produced by Sigma Corporation) was added such that the concentration thereof was 1 wt%, and it was incubated at 37°C for 1 hour so as to be blocked. After the thus blocked antibody sensitization latex particles were washed with a phosphate buffer solution, the latex particles were dispersed in a latex-dispersed buffer solution (in 50 mM Tris-HCl buffer solution (pH 8.4), 0.1 wt% BSA, 0.1 wt% NaN₃), thereby preparing the antihemolytic streptococcus antibody sensitization latex reagent (particle concentration of 0.1 wt% (w/v)).

### 2. Measurement of hemolytic streptococcus antigen by latex agglutination assay

As an antigen, Picibanil (produced by Chugai Pharmaceutical Co., Ltd.), of which the Su strain of streptococcus pyogenes (Group A Type 3) was treated with penicillin, was used. The antigen was diluted with a specimen diluent (PBS (pH 7.4) containing 1 wt% BSA and 0.1 wt% NaN₃) so as to prepare antigen solutions of two kinds of antigen concentrations (10 ng/mL and 50 ng/mL). Thereafter, each of the antigen solutions of the respective concentrations was mixed with the 2 M sodium nitrite solution and the 1 M acetic acid solution immediately before the measurement, and subsequently was neutralized, thereby preparing the specimen solution. Herein, the specimen solution with the antigen concentration of 0 ng/mL was prepared by mixing the specimen diluent, the 2 M sodium nitrite solution and the 1 M acetic acid solution, and subsequently neutralizing them.

63 µL of the antibody sensitization latex reagent, 14 µL of the specimen solution and 63 µL of a reaction buffer solution were mixed in a cuvette, and an absorbance change (measurement wavelength of 660 nm) was measured at 37°C for 5 minutes by using an immunoreaction measuring system (product name: SPOTCHEM-IM, produced by Arkray, Inc.). Results thereof will be shown in Table 1 below as an absorbance change for 5 minutes (absorbance after 5 minutes from starting of the measurement - absorbance immediately after the starting of the measurement). It should be noted that the reaction buffer solution having a below-described composition was used.

### (Reaction buffer solution)

| | |
|---|---|
| 1.9 wt% | polyethylene glycol 20000 (produced by NACALAI TESQUE, INC.) |
| 1 wt% | BSA |
| 2 wt% | NaCl |
| 0.1 wt% | EDTA-2Na |
| 0.1 wt% | NaN₃ |
| 200 mM | Tris-HCl buffer solution (pH 8.4) |

**[Table 1]**

| antigen concentration (ng/mL) | absorbance change (660nm) |
|---|---|
| 0 | 0.0010 |
| 10 | 0.0070 |
| 50 | 0.0310 |

As shown in Table 1 above, the absorbance change was increased according to the antigen concentration of the hemolytic streptococcus, and it could be confirmed that the amount of the hemolytic streptococcus can be measured quantitatively by the latex agglutination assay.

### 3. Measurement of hemolytic streptococcus negative specimen

A throat swab was collected from a person who clearly was not infected with a hemolytic streptococcus, judged from his/her symptom. The throat swab was checked to be bacteriolytic streptococcus negative, by using a commercially available bacteriolytic streptococcus detecting kit (product name: Strep A Testpack; produced by Plusbot Japan Inc.). Thereafter, a throat swab was collected from the person who was checked to be negative by using a cotton swab, and an amount of the bacteriolytic streptococcus was measured under Condition 1 described below. Moreover, as Comparative Example 1, an amount of the bacteriolytic streptococcus of the specimen was measured under Condition 2 described below.

### (Condition 1)

The throat swab, the 2 M sodium nitrite solution and the 1 M acetic acid solution were mixed, and subsequently were neutralized so as to prepare a specimen solution. Thereafter, an extraction unit, in which an ion-exchange membrane disk filter (PALL/ produced by Nihon Pall Ltd., product name: I. C. E450 (registered trademark) (cation exchange resin: polyethersulfone, functional group: sulfonic acid group, mesh size of filter: 0.45 µm)) with a thickness of 140 µm was disposed between glass filters, was filled in a nozzle that was formed of polyethylene. The specimen solution was poured into a polyethylene tube for treating the specimen, and subsequently, the nozzle was attached to the tube. Thereafter, the specimen solution was passed through the nozzle and was filtered thereby so as to be subjected to cation exchange treatment. Similarly to the above-described "2. Measurement of hemolytic streptococcus antigen by latex agglutination assay", an absorbance change at a measurement wavelength of 660 nm was measured by using the specimen solution after the cation exchange treatment.

### (Comparative Example 1; Condition 2)

The throat swab, the 2 M sodium nitrite solution and the 1 M acetic acid solution were mixed, and subsequently were neutralized, which was used directly so as to measure an absorbance change similarly to Condition 1 described above.

Measurement results of the above-described Example 1 (Condition 1) and Comparative Example 1 (Condition 2) will be shown in Table 2 below. It should be noted that, in the table, in the case where the absorbance change was 0.0050 or more, the result was decided to be positive (+), and in the case where the absorbance change was less than 0.0050, the result was decided to be negative (-).

**[Table 2]**

| specimen number | Example 1 (Condition 1) | | Comparative Example 1 (Condition 2) | |
|---|---|---|---|---|
| | absorbance change | decision | absorbance change | decision |
| 1 | 0.0012 | - | 0.0203 | + |
| 2 | 0.0027 | - | 0.0063 | + |
| 3 | 0.0032 | - | 0.0107 | + |
| 4 | 0.0016 | - | 0.0054 | + |
| 5 | 0.0021 | - | 0.0044 | - |
| 6 | 0.0025 | - | 0.0173 | + |
| 7 | 0.0021 | - | 0.0091 | + |
| 8 | 0.0023 | - | 0.0100 | + |
| 9 | 0.0023 | - | 0.0142 | + |
| 10 | 0.0015 | - | 0.0177 | + |
| 11 | 0.0029 | - | 0.0201 | + |
| 12 | 0.0020 | - | 0.0055 | + |
| 13 | 0.0020 | - | 0.0013 | - |
| 14 | 0.0029 | - | 0.0128 | + |

As shown in Table 2 above, in the measurement of the hemolytic streptococcus by the latex agglutination assay, in Comparative Example 1 (Condition 2) where the cation exchange treatment was not carried out, regardless of using the specimen that was bacteriolytic streptococcus negative, false positives occurred due to nonspecific agglutination reactions in twelve specimens among the fourteen specimens. On the other hand, in Example 1 (Condition 1) where the cation exchange treatment was carried out in advance, negative results that were correct were obtained from all of the fourteen specimens.

From these results, it was realized that the nonspecific reaction can be suppressed sufficiently by carrying out the cation exchange treatment with respect to the specimen in advance in the immune agglutination by the latex agglutination assay.

### Example 2

### 1. Preparation of antiinfluenza type A antibody sensitization latex reagent

An antibody solution (antibody concentration of 0.8 mg/mL) was prepared by using an antiinfluenza type A mouse monoclonal antibody in 50 mM phosphate buffer solution (pH 7.4). Similarly to the antihemolytic streptococcus antibody sensitization latex reagent in Example 1 above, the antiinfluenza type A antibody sensitization latex reagent was prepared by using the antibody solution.

### 2. Measurement of influenza type A antigen by latex agglutination assay

As the antigen, a purified influenza type A virus A/Kiev/301/94 (H3N2) was used. The antigen was diluted by using the specimen diluent that was used in Example 1 so as to prepare antigen solutions of two kinds of antigen concentrations (4 µg/mL and 20 µg/mL). Thereafter, immediately before the measurement, each of the antigen solutions of the respective concentrations was diluted ten times with a specimen extract solution with a composition described below, which was used as the specimen solution for the below-described measurements. Herein, the specimen solution having an antigen concentration of 0 ng/mL was prepared by diluting the specimen diluent ten times with the specimen extract solution.

### (Specimen extract solution)

| | |
|---|---|
| 0.4 mg/mL | semialkali protease |
| 1 wt% | n-Octanol-N-methylglucamide |
| 0.5 wt% | BSA |
| 0.9 wt% | NaCl |
| 0.1 wt% | EDTA-2Na |
| 50 mM | Tris-HCl buffer solution (pH 8.4) |

56 µL of the antibody sensitization latex reagent, 28 µL of the specimen solution and 56 µL of a reaction buffer solution were mixed in a cuvette, and an absorbance change at a measurement wavelength of 660 nm was measured, similarly to Example 1 described above. Results thereof will be shown in Table 3 below. Herein, the reaction buffer solution had a composition described below.

### (Reaction buffer solution)

| | |
|---|---|
| 2.1 wt% | polyethylene glycol 20000 (produced by NACALAI TESQUE, INC.) |
| 1 wt% | BSA |
| 0.9 wt% | NaCl |
| 0.1 wt% | EDTA-2Na |
| 0.1 wt% | NaN₃ |
| 200 mM | Tris-HCl buffer solution (pH 8.4) |

**[Table 3]**

| antigen concentration (µg/mL) | absorbance change (660 nm) |
|---|---|
| 0 | -0.0039 |
| 4 | 0.0344 |
| 20 | 0.1043 |

As shown in Table 3 described above, the absorbance change was increased according to the concentration of the influenza type A virus, and it could be confirmed that an amount of the influenza type A virus can be measured quantitatively by the latex agglutination assay.

### 3. Measurement of influenza type A virus negative specimen

A nasal aspirate was collected from a person who clearly was not infected with an influenza type A virus, judged from his/her symptom. The nasal aspirate was checked to be influenza type A virus negative, by using a commercially available influenza virus detecting kit (product name: Capilia FluA/B; produced by Nippon Becton, Dickinson and Company). The nasal aspirate was collected from the person who was checked to be negative and was suspended in 1 mL of the specimen extract solution by using a cotton swab (hereinafter, called a "suspension"). By using the suspension, the amount of the influenza type A virus was measured under Conditions 1 and 2 described below. Moreover, as Comparative Example 2, the amount of the influenza type A virus of the specimen was measured under Condition 3 described below.

### (Example 2-1: Condition 1)

The suspension was added into COSMOGEL (registered trademark) 30SP (product name) (cation exchange carrier (functional group: sulfonic acid group, particle diameter: 10 µm); produced by NACALAI TESQUE, INC.), and was let stand for 2 minutes so as to be subjected to cation exchange treatment. Subsequently, similarly to the above-described "2. Measurement of hemolytic streptococcus antigen by latex agglutination assay", an absorbance change at a measurement wavelength of 660 nm was measured.

### (Example 2-2: Condition 2)

The suspension was added into COSMOGEL (registered trademark) 30CM (product name) (cation exchange carrier (functional group: carboxyl group, particle diameter: 10 µm); produced by NACALAI TESQUE, INC.), and was let stand for 2 minutes so as to be subjected to cation exchange treatment. Subsequently, similarly to Condition 1 described above, an absorbance change was measured.

### (Comparative Example 2: Condition 3)

The suspension was used directly, and a change of absorbance thereof was measured similarly to Condition 1 described above.

Measurement results under the above-described Conditions 1 to 3 will be shown in Table 4 below. In the examples listed in the table, the specimen numbers 1 to 4 represent the measurement results of the specimens that were subjected to the cation exchange treatment by the method of Condition 1 (Example 2-1), and the specimen numbers 5 to 7 represent the measurement results of the specimens that were subjected to the cation exchange treatment by the method of Condition 2 (Example 2-2). In the table, in the case where the absorbance change was 0.0050 or more, the result was decided to be positive (+), and in the case where the absorbance change was less than 0.0050, the result was decided to be negative (-).

**[Table 4]**

| specimen number | Example 2 | | Comparative Example 2 | |
|---|---|---|---|---|
| | absorbance change | decision | absorbance change | decision |
| 1 | -0.0009 | - | 0.0556 | + |
| 2 | 0.0018 | - | 0.0536 | + |
| 3 | 0.0014 | - | 0.0569 | + |
| 4 | -0.0033 | - | 0.0582 | + |
| 5 | 0.0038 | - | 0.0992 | + |
| 6 | 0.0008 | - | 0.0536 | + |
| 7 | 0.0023 | - | 0.0536 | + |

As shown in Table 4 above, in the measurement of the influenza type A virus by the latex agglutination assay, in Comparative Example 2 (Condition 3) where the cation exchange treatment was not carried out, regardless of using the specimen that was influenza type A virus negative, false positives occurred due to nonspecific agglutination reactions in all seven specimens. On the other hand, in Example 2 (Conditions 1 and 2) where the cation exchange treatment was carried out, negative results that were correct were obtained from all of the specimens.

From these results, it was realized that the nonspecific reaction can be suppressed sufficiently by carrying out the cation exchange treatment with respect to the specimen in the immune agglutination by the latex agglutination assay.

It should be noted that, the above-described Examples 1 and 2 showed the examples where the measurement method of the present invention was carried out by using the specimens that were checked to be negative in advance, but according to the measurement method of the present invention, it was realized that a positive reaction can be detected from a positive specimen.

### Industrial Applicability

As described above, according to the immunoassay method and the immunoassay kit of the present invention, since a nonspecific reaction can be prevented, an immunoassay method with a high detecting precision and a high quantification precision can be achieved without occurrence of false positives. Thus, the immunoassay method of the present invention can be applied to wide range of fields in, for example, medical and biological science, and is particularly useful in a field of a clinical examination among them.

## Claims

1. An immunoassay method for detecting or quantifying an analyte in a specimen, comprising:
a step of mixing the specimen with an antibody sensitization particle obtained by sensitizing a particle with an antibody that reacts with the analyte in the specimen;
a step of measuring agglutination of the antibody sensitization particle by an antigen-antibody reaction; and
a step of pre-treating the specimen by using a cation exchange resin having a sulfonic acid group as the functional group in advance of the mixing step,
wherein the analyte is a bacterium and/or a virus.

2. The immunoassay method according to claim 1, wherein the cation exchange resin is at least one of a strong acid cation exchange material and a weak acid cation exchange material.

3. The immunoassay method according to claim 1, wherein the cation exchange resin is at lease one selected from: polyethersulfone; polysulfone; a resin obtained by introducing a sulfonic acid group into a polystyrene-divinylbenzene copolymer; and a resin obtained by introducing a sulfonic acid group into a hydrophilic methacrylate-based polymer.

4. The immunoassay method according to claim 1, wherein a form of the cation exchange resin is at least one of a cation exchange filter and a cation exchange column.

5. The immunoassay method according to claim 4, wherein the cation exchange filter is a filter in which a spherical-shaped cation exchange resin is supported by a carrier or a filter containing a fiber-shaped cation exchange resin.

6. The immunoassay method according to claim 1, wherein the antibody is at least one of a polyclonal antibody and a monoclonal antibody.

7. The immunoassay method according to claim 1, wherein the antibody sensitization particle is an antibody sensitization particle that is obtained by sensitizing an insoluble carrier particle with an antibody.

8. The immunoassay method according to claim 7, wherein the insoluble carrier particle is a latex particle.

9. The immunoassay method according to claim 1, wherein the analyte is at least one selected from the group consisting of: a hemolytic streptococcus; an influenza virus; an adenovirus; a RS virus; a Hemophilus pertussis; a chlamydia trachomatous; a coronavirus; and a mycoplasma.

10. An immunoassay kit used in the immunoassay method according to claim 1, comprising: an antibody sensitization particle obtained by sensitizing with an antibody that reacts with a bacterium and/or a virus in a specimen; and a cation exchange resin, having a sulfonic acid group as the functional group, wherein a form of the cation exchange resin is at least one of a cation exchange filter and a cation exchange column.

11. The immunoassay kit according to claim 10, wherein the cation exchange resin is at least one selected from: polyethersulfone; polysulfone; a resin obtained by introducing a sulfonic acid group into a polystyrene-divinylbenzene copolymer; and a resin obtained by introducing a sulfonic acid group into a hydrophilic methacrylate-based polymer.

12. The immunoassay kit according to claim 10, wherein the antibody sensitization particle is an antibody sensitization particle that is obtained by sensitizing an insoluble carrier particle with an antibody.

13. The immunoassay kit according to claim 12, wherein the insoluble carrier particle is a latex particle.

14. The immunoassay kit according to claim 10, further comprising a reagent pack containing a reagent, wherein the reagent comprises the antibody sensitization particle.

15. The immunoassay kit according to claim 14, wherein the reagent pack has disposable cartridge form.

16. The kit according to claim 10, further comprising at least one selected from the group consisting of: a specimen diluent; a reaction buffer solution; a specimen collecting tool; and a specimen extract solution.

## Patentansprüche

1. Immunassay-Verfahren zur Bestimmung oder Quantifizierung eines Analyten in einer Probe, das umfasst:
einen Schritt, die Probe mit einem Antikörpersensibilisierungspartikel zu mischen, der durch Sensibilisieren eines Partikels mit einem Antikörper erhalten wird, welcher mit dem Analyten in der Probe reagiert;
einen Schritt, eine Agglutination des Antikörpersensibilisierungspartikels durch eine Antigen-Antikörperreaktion zu messen; und
einen Schritt, die Probe durch Verwenden eines Kationenaustauschharzes mit einer Sulfonsäuregruppe als funktioneller Gruppe vor dem Mischschritt vorzubehandeln,
wobei der Analyt ein Bakterium und/oder eine Virus ist.

2. Immunassay-Verfahren nach Anspruch 1, bei dem das Kationenaustauschharz ein starkes Säure-Kationenaustauschermaterial und/oder ein schwaches Säure-Kationenaustauschermaterial ist.

3. Immunassay-Verfahren nach Anspruch 1, bei dem das Kationen-Austauscherharz wenigstens eines der Folgenden umfasst: Polyethersulfon; Polysulfon; ein Harz, das durch Einführen einer Sulfonsäuregruppe in ein Polystyrol-Divinylbenzol-Copolymer erhalten wird; und ein Harz, das durch Einführen einer Sulfonsäuregruppe in ein hydrophiles methacrylatbasiertes Polymer erhalten wird.

4. Immunassay-Verfahren nach Anspruch 1, bei dem eine Form des Kationenaustauscherharzes ein Kationenaustauscherfilter und/oder eine Kationenaustauschersäule ist.

5. Immunassay-Verfahren nach Anspruch 4, bei dem der Kationenaustauscherfilter ein Filter ist, in dem ein kugelförmiges Kationenaustauscherharz durch einen Träger oder einen Filter getragen wird, der ein faserförmiges Kationenaustauscherharz enthält.

6. Immunassay-Verfahren nach Anspruch 1, bei dem der Antikörper ein polyklonaler und/oder monoklonaler Antikörper ist.

7. Immunassay-Verfahren nach Anspruch 1, bei dem der Antikörpersensibilisierungspartikel ein Antikörpersensibilisierungspartikel ist, der durch Sensibilisieren eines unlöslichen Trägerpartikels mit einem Antikörper erhalten wird.

8. Immunassay-Verfahren nach Anspruch 7, bei dem der unlösliche Trägerpartikel ein Latexpartikel ist.

9. Immunassay-Verfahren nach Anspruch 1, bei dem der Analyt wenigstens einer ist, der aus der Gruppe ausgewählt ist, die besteht aus: einem hämolytischen Streptococcus; einem Influenzavirus; einem Adenovirus; einem RS-Virus; einem Hämophilus Pertussis; einer Chlamydia trachomatis; einem Coronavirus; und einem Mycoplasma.

10. Immunassay-Kit, der in dem Immunassay-Verfahren nach Anspruch 1 verwendet wird, der umfasst: einen Antikörpersensibilisierungspartikel, der durch Sensibilisieren mit einem Antikörper erhalten wird, der mit einem Bakterium und/oder einem Virus in einer Probe reagiert; und einem Kationenaustauscherharz mit einer Sulfonsäuregruppe als funktioneller Gruppe, wobei eine Form des Kationenaustauschers ein Kationenaustauscherfilter und/oder eine Kationenaustauschersäule ist.

11. Immunassay-Kit nach Anspruch 10, bei dem das Kationenaustauscherharz wenigstens eines der Folgenden ist: Polyethersulfon; Polysulfon; ein Harz, das durch Einführen einer Sulfonsäuregruppe in ein Polystyrol-Divinylbenzol-Copolymer erhalten wird; und ein Harz, das durch Einführen einer Sulfonsäuregruppe in ein hydrophiles methacrylatbasiertes Polymer erhalten wird.

12. Immunassay-Kit nach Anspruch 10, bei dem der Antikörpersensibilisierungspartikel ein Antikörpersensibilisierungspartikel ist, der durch Sensibilisieren eines unlöslichen Trägerpartikels mit einem Antikörper erhalten wird.

13. Immunassay-Kit nach Anspruch 12, bei dem der unlösliche Trägerpartikel ein Latexpartikel ist.

14. Immunassay-Kit nach Anspruch 10, der ferner ein Reagenzienpaket mit einem Reagenz aufweist, wobei das Reagenz den Antikörpersensibilisierungspartikel enthält.

15. Immunassay-Kit nach Anspruch 14, bei dem das Reagenzpaket eine wegwerfbare Cartridge-Form aufweist.

16. Kit nach Anspruch 10, der ferner wenigstens eines aus der Gruppe aufweist, die besteht aus: einem Probenverdünnungsmittel; einer Reaktionspufferlösung; einem Probenauffanggerät; und einer Probenextraktlösung.

## Revendications

1. Procédé de dosage immunologique pour détecter ou quantifier un analyte dans un échantillon, comprenant :
une étape de mélange de l'échantillon avec une particule de sensibilisation aux anticorps, obtenue en sensibilisant une particule avec un anticorps réagissant avec l'analyte dans l'échantillon ;
une étape de mesure de l'agglutination de la particule de sensibilisation aux anticorps par une réaction antigène-anticorps ; et
une étape de prétraitement de l'échantillon en utilisant une résine échangeuse de cations comprenant un groupe acide sulfonique comme groupe fonctionnel avant l'étape de mélange,
dans lequel l'analyte est une bactérie et/ou un virus.

2. Procédé de dosage immunologique selon la revendication 1, dans lequel la résine échangeuse de cations est au moins une résine parmi une matière échangeuse de cations d'acide fort et une matière échangeuse de cations d'acide faible.

3. Procédé de dosage immunologique selon la revendication 1, dans lequel la résine échangeuse de cations est au moins une résine choisie parmi : une polyéthersulfone ; une polysulfone ; une résine obtenue en introduisant un groupe acide sulfonique dans un copolymère polystyrène-divinylbenzène ; et une résine obtenue en introduisant un groupe acide sulfonique dans un polymère hydrophile à base de méthacrylate.

4. Procédé de dosage immunologique selon la revendication 1, dans lequel une forme de la résine échangeuse de cations est au moins une forme parmi un filtre échangeur de cations et une colonne échangeuse de cations.

5. Procédé de dosage immunologique selon la revendication 4, dans lequel le filtre échangeur de cations est un filtre dans lequel une résine échangeuse de cations de forme sphérique est supportée par un transporteur ou un filtre contenant une résine échangeuse de cations sous forme de fibres.

6. Procédé de dosage immunologique selon la revendication 1, dans lequel l'anticorps est au moins un anticorps parmi un anticorps polyclonal et un anticorps monoclonal.

7. Procédé de dosage immunologique selon la revendication 1, dans lequel la particule de sensibilisation aux anticorps est une particule de sensibilisation aux anticorps qui est obtenue en sensibilisant une particule de transporteur insoluble avec un anticorps.

8. Procédé de dosage immunologique selon la revendication 7, dans lequel la particule de transporteur insoluble est une particule de latex.

9. Procédé de dosage immunologique selon la revendication 1, dans lequel l'analyte est au moins un analyte choisi dans le groupe comprenant : un streptocoque hémolytique ; un virus de la grippe ; un adénovirus ; un virus RS ; une Hemophilus pertussis ; une chlamydia trachomatis ; un coronavirus ; et un mycoplasme.

10. Kit de dosage immunologique utilisé dans le procédé de dosage immunologique selon la revendication 1, comprenant : une particule de sensibilisation aux anticorps obtenue en sensibilisant la particule avec un anticorps réagissant avec une bactérie et/ou un virus dans un échantillon ; et une résine échangeuse de cations ; comprenant un groupe acide sulfonique comme groupe fonctionnel, dans lequel une forme de la résine échangeuse de cations est au moins une forme parmi un filtre échangeur de cations et une colonne échangeuse de cations.

11. Kit de dosage immunologique selon la revendication 10, dans lequel la résine échangeuse de cations est au moins une résine choisie parmi : une polyéthersulfone ; une polysulfone ; une résine obtenue en introduisant un groupe acide sulfonique dans un copolymère polystyrène-divinylbenzène ; et une résine obtenue en introduisant un groupe acide sulfonique dans un polymère hydrophile à base de méthacrylate.

12. Kit de dosage immunologique selon la revendication 10, dans lequel la particule de sensibilisation aux anticorps est une particule de sensibilisation aux anticorps qui est obtenue en sensibilisant une particule de transporteur insoluble avec un anticorps.

13. Kit de dosage immunologique selon la revendication 12, dans lequel la particule de transporteur insoluble est une particule de latex.

14. Kit de dosage immunologique selon la revendication 10, comprenant en outre un pack réactif contenant un réactif, dans lequel le réactif comprend la particule de sensibilisation aux anticorps.

15. Kit de dosage immunologique selon la revendication 14, dans lequel le pack réactif comprend une forme de cartouche jetable.

16. Kit selon la revendication 10, comprenant en outre au moins un élément choisi dans le groupe comprenant : un diluant de l'échantillon ; une solution tampon de réaction ; un outil de collecte de l'échantillon ; et une solution d'extraction de l'échantillon.
